# EUROPEAN PATENT APPLICATION

(11) **EP 4 606 904 A1**
(43) Date of publication of application: **27.08.2025**
(21) Application number: 23878548.9
(22) Date of filing: 22.03.2023
(51) Int. Cl.: C12P 19/38, C12P 19/40, C12N 9/10

(54) **METHOD FOR BIOSYNTHESIS OF ARABINONUCLEOSIDE BY ONE-POT METHOD, AND COMPOSITION**

(30) Priority: 21.10.2022 CN 202211296616
(71) Applicant: Asymchem Life Science (Tianjin) Co., Ltd, Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); JIAO, Xuecheng, Tianjin 300457 (CN); LI, Mingji, Tianjin 300457 (CN); LI, Rui, Tianjin 300457 (CN); DING, Shimao, Tianjin 300457 (CN)
(74) Representative: Zacco GmbH
(86) International application number: PCT/CN2023/083184
(87) International publication number: WO 2024/082546

(57) **Abstract**

The present application provides a method for one-pot biosynthesis of arabinonucleoside, and a composition. The method for one-pot biosynthesis of arabinonucleoside includes: mixing a substrate, uracil nucleoside phosphorylase or pyrimidine nucleoside phosphorylase, and purine nucleoside phosphorylase together, then performing biosynthesis, and directly obtaining arabinonucleoside through preparation by means of a one-pot method. The uracil nucleoside phosphorylase includes UP shown in SEQ ID NO: 1; the pyrimidine nucleoside phosphorylase includes PyNP shown in SEQ ID NO: 2; the purine nucleoside phosphorylase includes PNP shown in SEQ ID NO: 3; the substrate includes 1-beta-D-Arabinofuranosyluracil and a substrate base. Therefore, the problem in the prior art of complex operations for biosynthesis two-step preparation of arabinonucleoside can be solved, and the present application is applicable to the field of enzyme catalysis.

## Description

This application is based upon and claims priority to Chinese Patent Application No. 202211296616.9 filed on October 21, 2022, the application of which is hereby incorporated by reference in its entirety.

### Technical Field

The present application relates to the field of enzyme catalysis, and specifically, to a method for one-pot biosynthesis of arabinonucleoside, and a composition.

### Background

Arabinonucleoside is nucleoside that replaces deoxyribose with arabinose. When incorporated into DNA, arabinonucleoside can block DNA replication, thereby affecting cell division and proliferation. As a result, arabinonucleoside may be used as an antiviral and anti-tumor drug to treat related diseases, such as herpes, tumors, AIDS, hepatitis B, etc. At present, arabinonucleoside is mainly synthesized by chemical methods (CN1042939C, CN107556356A, CN1128270A, CN103467468A, and CN107892707A) and biological methods (CN106929553A, JPH10286097A, and CN105237602A). The chemical synthesis process is complex in process, high in cost, and strict in reaction condition, and needs to use heavy metals, organic solvents, etc., resulting in hazards to the health of personnel and environment pollution. The biological method uses enzymes expressed by microorganisms as catalysts to convert substrates into relevant products under mild conditions, with simple operating procedures and relatively mild reaction conditions, without the use of substances such as heavy metals, organic reagents, etc. harmful to human bodies and environments.

The patent application CN106929553A discloses a method for two-step synthesis of vidarabine. 1-beta-D-Arabinofuranosyluracil may be converted into arabinose-1-phosphate with uracil nucleoside phosphorylase. After separation, vidarabine is synthesized from arabinose-1-phosphate and adenine through the action of adenosine phosphorylase, with a conversion rate over 90%. Wei Xiaokun et al. published a method for two-step synthesis of guaninearabinoside (Ara-G). First, *Enterobacter Aerogenes,* which expresses purine nucleoside phosphorylase and pyrimidine nucleoside phosphorylase, is used to synthesize 1-beta-D-Arabinofuranosyluracil and 2,6-diaminopurine into 2,6-Diamino-9-(beta-D-arabinofuranosyl)purine. Then adenosine deaminase derived from *Aspergillus oryzae* is used to deaminate 2,6-Diamino-9-(beta-D-arabinofuranosyl)purine, thereby yielding Ara-G. When a concentration of a substrate is lower than 10 mM, a conversion rate of the 2,6-Diamino-9-(beta-D-arabinofuranosyl)purine is 80%. However, when the concentration of the substrate is higher than 10 mM, the conversion rate of the 2,6-Diamino-9-(beta-D-arabinofuranosyl)purine is significantly reduced to less than 50%.

### Summary

The present application is mainly intended to provide a method for one-pot biosynthesis of arabinonucleoside, and a composition, so as to solve the problem in the prior art of complex operations for biosynthesis two-step preparation of arabinonucleoside.

In order to implement the above objective, a first aspect of the present application provides a method for one-pot biosynthesis of arabinonucleoside. The method includes: a substrate, a purine nucleoside phosphorylase and any one of the following together: a uracil nucleoside phosphorylase or a pyrimidine nucleoside phosphorylase, are mixed together, then biosynthesis is performed, and arabinonucleoside is directly obtained through preparation by means of a one-pot method. The uracil nucleoside phosphorylase includes UP, or a protein that is of more than 80% identity to the UP and has a function same as the UP, and the UP is a protein shown in SEQ ID NO: 1; the pyrimidine nucleoside phosphorylase includes PyNP, or a protein that is of more than 80% identity to the PyNP and has a function same as the PyNP, and the PyNP is a protein shown in SEQ ID NO: 2; the purine nucleoside phosphorylase includes PNP, or a protein that is of more than 80% identity to the PNP and has a function same as the PNP, and the PNP is a protein shown in SEQ ID NO: 3; and the substrate includes 1-beta-D-Arabinofuranosyluracil, a substrate base, and a substrate phosphate.

Further, the method includes: arabinose-1-phosphate and free bases are generated by the 1-beta-D-Arabinofuranosyluracil and the substrate phosphate under the catalysis of the uracil nucleoside phosphorylase or the pyrimidine nucleoside phosphorylase; and the arabinose-1-phosphate is catalyzed with the purine nucleoside phosphorylase, and a phosphate group is substituted with the substrate base, so as to obtain the arabinonucleoside.

Further, the substrate base is a base shown in Formula I, where R₁ is selected from -NH₂, =O, -OMe or -Cl, and R₂ is selected from -H, -NH₂, -Cl or -F; and preferably, the substrate base includes 2,6-diaminopurine, 2-amino-6-methoxypurine, 2-chloroadenine or 2-fluoroadenine.

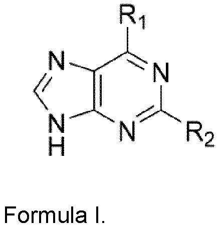

Further, one or more of the purine nucleoside phosphorylase, the uracil nucleoside phosphorylase or the pyrimidine nucleoside phosphorylase are purified proteins, crude enzyme liquid or immobilized enzymes.

Further, a catalytic time for the one-pot biosynthesis is 4-20 h; preferably, a catalytic temperature of the one-pot biosynthesis is 50-80 °C, more preferably 60-70 °C; and preferably, a concentration of the 1-beta-D-Arabinofuranosyluracil is 2-320 mM, a concentration of the substrate base is 1-200 mM, and a concentration of the substrate phosphate is 1-100 mM.

Further, the arabinonucleoside includes 2,6-Diamino-9-(beta-D-arabinofuranosyl)purine, nelarabine, 2-chloroadeninearabinoside, or fludarabine; and the substrate phosphate includes one or more of sodium monohydrogen phosphate, sodium dihydrogen phosphate, potassium monohydrogen phosphate, or potassium dihydrogen phosphate, or a phosphate buffer, or a phosphate buffer solution.

In order to implement the above objective, a second aspect of the present application provides a composition. The composition includes a purine nucleoside phosphorylase and any one of the following: a uracil nucleoside phosphorylase or a pyrimidine nucleoside phosphorylase. The uracil nucleoside phosphorylase includes UP, or a protein that is of more than 80% identity to the UP and has a function same as the UP, and the UP is a protein shown in SEQ ID NO: 1; the pyrimidine nucleoside phosphorylase includes PyNP, or a protein that is of more than 80% identity to the PyNP and has a function same as the PyNP, and the PyNP is a protein shown in SEQ ID NO: 2; and the purine nucleoside phosphorylase includes PNP, or a protein that is of more than 80% identity to the PNP and has a function same as the PNP, and the PNP is a protein shown in SEQ ID NO: 3.

Further, one or more of the purine nucleoside phosphorylase, the uracil nucleoside phosphorylase or the pyrimidine nucleoside phosphorylase are purified proteins, crude enzyme liquid or immobilized enzymes.

Further, the composition further includes 1-beta-D-Arabinofuranosyluracil, a substrate base, and a substrate phosphate; and the substrate phosphate includes one or more of sodium monohydrogen phosphate, sodium dihydrogen phosphate, potassium monohydrogen phosphate, or potassium dihydrogen phosphate, or a phosphate buffer, or a phosphate buffer solution.

Further, the substrate base is a base shown in Formula I, where R₁ is selected from -NH₂, =O, -OMe or -Cl, and R₂ is selected from -H, -NH₂, -Cl or -F; preferably, the substrate base includes 2,6-diaminopurine, 2-amino-6-methoxypurine, 2-chloroadenine or 2-fluoroadenine; and preferably, a concentration of the 1-beta-D-Arabinofuranosyluracil is 2-320 mM, a concentration of the substrate base is 1-200 mM, and a concentration of the substrate phosphate is 1-100 mM.

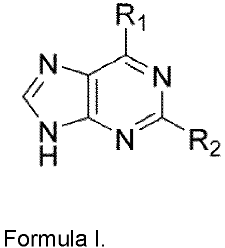

Through the application of the technical solutions of the present application, with the purine nucleoside phosphorylase, and the pyrimidine nucleoside phosphorylase or the uracil nucleoside phosphorylase, the 1-beta-D-Arabinofuranosyluracil and the substrate base can be used as substrates, a target product arabinonucleoside is prepared through one-pot biosynthesis, and operations of midway material replenishment or intermediate product purification and re-feeding, etc., are not required, such that compared to two-step preparation, simpler operations are achieved, and scale-up production is realized with industrialization.

### Brief Description of the Drawings

The drawings, which form a part of the present application, are used to provide a further understanding of the present application. The exemplary embodiments of the present application and the description thereof are used to explain the present application, but do not constitute improper limitations to the present application. In the drawings:
Fig. 1 is an HPLC diagram of synthesis of 2,6-Diamino-9-(beta-D-arabinofuranosyl)purine with 1-beta-D-Arabinofuranosyluracil and 2,6-diaminopurine as substrates according to Embodiment 2 of the present application.
Fig. 2 is an HPLC diagram of enlarged synthesis of 2,6-Diamino-9-(beta-D-arabinofuranosyl)purine with 1-beta-D-Arabinofuranosyluracil and 2,6-diaminopurine as substrates according to Embodiment 3 of the present application.
Fig. 3 is an HPLC diagram of synthesis of nelarabine with 1-beta-D-Arabinofuranosyluracil and 2-amino-6-methoxypurine as substrates according to Embodiment 4 of the present application.
Fig. 4 is an HPLC diagram of enlarged synthesis of nelarabine with 1-beta-D-Arabinofuranosyluracil and 2-amino-6-methoxypurine as substrates according to Embodiment 5 of the present application.
Fig. 5 is an HPLC diagram of synthesis of 2-chloroadeninearabinoside with 1-beta-D-Arabinofuranosyluracil and 2-chloroadenine as substrates according to Embodiment 6 of the present application.
Fig. 6 is an HPLC diagram of synthesis of fludarabine with 1-beta-D-Arabinofuranosyluracil and 2-chloroadenine as substrates according to Embodiment 7 of the present application.

### Detailed Description of the Embodiments

It is to be noted that the embodiments in the present application and the features in the embodiments may be combined with one another without conflict. The present application will be described below in detail with reference to the embodiments.

As mentioned in the Background, biosynthesis of vidarabine in the prior art mostly uses a two-step method, which is tedious in operation, long in required time, and not conductive to industrialized mass production. Therefore, the inventor in the present application attempts to explore a method for one-pot biosynthesis of arabinonucleoside, thereby proposing a series of protective solutions of the present application.

A typical implementation of the present application provides a method for one-pot biosynthesis of arabinonucleoside. The method includes: a substrate, a purine nucleoside phosphorylase and any one of the following together: a uracil nucleoside phosphorylase or a pyrimidine nucleoside phosphorylase are mixed together, then biosynthesis is performed, and arabinonucleoside is directly obtained through preparation by means of a one-pot method. The uracil nucleoside phosphorylase includes UP, or a protein that is of more than 80% identity to the UP and has a function same as the UP, and the UP is a protein shown in SEQ ID NO: 1; the pyrimidine nucleoside phosphorylase includes PyNP, or a protein that is of more than 80% identity to the PyNP and has a function same as the PyNP, and the PyNP is a protein shown in SEQ ID NO: 2; the purine nucleoside phosphorylase includes PNP, or a protein that is of more than 80% identity to the PNP and has a function same as the PNP, and the PNP is a protein shown in SEQ ID NO: 3; and the substrate includes a 1-beta-D-Arabinofuranosyluracil and a substrate base, and may further include a substrate phosphate to provide a phosphate group required by the reaction. The arabinonucleoside is nucleoside consisting of an arabinose structure and a base structure.

The uracil nucleoside phosphorylase used in the present application is a protein that is derived from *Trypanosoma cruzi,* shown in SEQ ID NO: 1, and named UP. The pyrimidine nucleoside phosphorylase is a protein that is derived from *Thermus thermophilus,* shown in SEQ ID NO: 2, and named PyNP. The purine nucleoside phosphorylase is a protein that is derived from *Geobacillus stearothermophilus,* shown in SEQ ID NO: 3, and named PNP. With the purine nucleoside phosphorylase and any one of the following enzymes: the uracil nucleoside phosphorylase or the pyrimidine nucleoside phosphorylase, one-pot biosynthesis can be performed with the 1-beta-D-Arabinofuranosyluracil and the substrate base as the substrates, the target product arabinonucleoside can be directly obtained without purifying an intermediate product before conversion. The uracil nucleoside phosphorylase, the pyrimidine nucleoside phosphorylase, or the purine nucleoside phosphorylase includes proteins that are of more than 80%, 85%, 90%, 95%, 98%, 99%, 99.5%, or 99.9% identity to the UP, PyNP, or PNP and have functions (i.e., the same catalytic functions) same as the UP, PyNP, or PNP, respectively.

As used herein, the amino acid residues are abbreviated below: Alanine (Ala; A), Asparagine (Asn; N), Aspartic acid (Asp; D), Arginine (Arg; R), Cysteine (Cys; C), Glutamate (Glu; E), Glutamine (Gln; Q), Glycine (Gly; G), Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V).

For rules of substitution, replacement, etc., if amino acids are similar in nature, replacements have similar effects. For example, in the homologous protein, conservative amino acid replacements may occur. "Conservative amino acid replacement" includes, but is not limited to:
Hydrophobic amino acids (Ala, Cys, Gly, Pro, Met, Val, Ile, and Leu) are substituted by other hydrophobic amino acids.
Hydrophobic amino acids with bulky side chains (Phe, Tyr, and Trp) are substituted by other hydrophobic amino acids with bulky side chains.
Amino acids with positively charged side chains (Arg, His, and Lys) are substituted by other amino acids with positively charged side chains.
Amino acids with polarized and uncharged side chains (Ser, Thr, Asn, and Gln) are substituted by other amino acids with polarized and uncharged side chains.

Person skilled in the art may also make conservative substitutions of the amino acids according to amino acid substitution rules known to those skilled in the art, such as a "blosum62 scoring matrix" in the prior art.

In a preferred embodiment, the method includes: arabinose-1-phosphate and free bases are generated by the 1-beta-D-Arabinofuranosyluracil and the substrate phosphate under the catalysis of the pyrimidine nucleoside phosphorylase or the uracil nucleoside phosphorylase; and the arabinose-1-phosphate is catalyzed with the purine nucleoside phosphorylase, and a phosphate group is substituted with the substrate base, so as to obtain the arabinonucleoside.

A reaction route of the method is as follows.

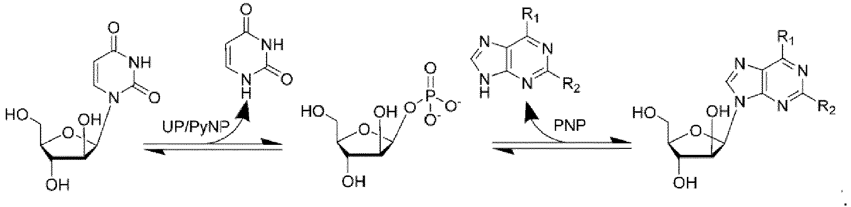

First, the substrate 1-beta-D-Arabinofuranosyluracil is catalyzed by the uracil nucleoside phosphorylase or the pyrimidine nucleoside phosphorylase, where the phosphate group derived from the substrate phosphate replaces the base portion of 1-beta-D-Arabinofuranosyluracil linked to the arabinose structure, forming phosphorylated arabinose and uracil. Then, under the catalysis of the purine nucleoside phosphorylase, the substrate base substitutes the phosphate group on the phosphorylated arabinose, so as to obtain the arabinonucleoside.

In the prior art, the two-step method is generally used to synthesize the arabinonucleoside, affecting the yield of the one-pot method. In the present application, enzymes with similar functions are screened to obtain a combination of the above enzymes. With the combination of the above enzymes, the arabinonucleoside can be prepared through the one-pot method, thereby improving a conversion rate of the substrate and the yield of the target product arabinonucleoside. The conversion rate of the method for one-pot biosynthesis is equivalent to that of the two-step method in the prior art, but is simpler in reaction operation, does not need to perform separation and purification on the intermediate product, is easy to achieve large-scale preparation in industrial production, and can reduce the time and cost required by the reaction.

In a preferred embodiment, the substrate base is a base shown in Formula I, where R₁ is selected from -NH₂, =O, -OMe or -Cl, and R₂ is selected from -H, -NH₂, -Cl or -F; and preferably, the substrate base includes 2,6-diaminopurine (i.e., 2-aminoadenine), 2-amino-6-methoxypurine, 2-chloroadenine or 2-fluoroadenine.

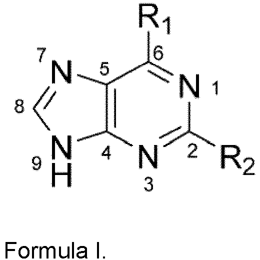

In a preferred embodiment, one or more of the purine nucleoside phosphorylase, the uracil nucleoside phosphorylase or the pyrimidine nucleoside phosphorylase are purified proteins, crude enzyme liquid or immobilized enzymes.

In a catalytic reaction, the above 3 enzymes may be present in various forms of the purified proteins, the crude enzyme liquid or the immobilized enzymes, and can all be catalyzed and synthesized into the nucleoside containing the protecting group. A gene expressing the PyNP and/or the UP and/or the PNP is cloned in a host cell, and after protein expression is induced, crude enzyme liquid containing a target protein can be obtained by breaking the host cell. The crude enzyme liquid is simple to prepare, has good catalytic capability, and can reduce the production cost of the catalytic reaction.

In a preferred embodiment, a catalytic time for enzyme catalysis is 4-20 h; preferably, a catalytic temperature of enzyme catalysis is 50-80 °C, more preferably, 60-70 °C; and preferably, a concentration of the 1-beta-D-Arabinofuranosyluracil is 2-320 mM, a concentration of the substrate base is 1-200 mM, and a concentration of the substrate phosphate is 1-100 mM. The substrate phosphate is not limited to a specific type, and includes, but is not limited to, phosphates commonly used in the prior art, including, but not limited to, one or more of sodium monohydrogen phosphate, sodium dihydrogen phosphate, potassium monohydrogen phosphate, or potassium dihydrogen phosphate, or mixtures such as Phosphate Buffer (PB), Phosphate Buffer Solution (PBS), or the like commercially available in the market. The PB includes a buffer consisting of a certain concentration of the sodium dihydrogen phosphate and disodium hydrogen phosphate. The PBS includes a buffer consisting of the disodium hydrogen phosphate, the potassium dihydrogen phosphate, and salts such as sodium chloride and potassium chloride.

In the above method, an amplification reaction can be performed, and in the reaction system, the concentration of the 1-beta-D-Arabinofuranosyluracil includes 2 mM, 10 mM, 20 mM, 50 mM, 100 mM, 200 mM, and can reach 320mM or more; and the concentration of the substrate base includes 1 mM, 2 mM, 10 mM, 20 mM, 50 mM, 100 mM, and can reach 200 mM or more, so as to perform large-scale preparation of the arabinonucleoside.

Within the above appropriate catalytic temperature and catalytic time, the enzyme catalysis reaction can be completed, and a conversion rate of the substrate base, i.e., the reaction yield, is higher. Enzymes or other reagents are not required to be replenished in the midway of the reaction, and subsequent catalysis is not required after the intermediate product is separated and purified. The reaction can be completed with one-pot catalysis, which is suitable for application in industrial scale-up production. The reaction conditions are mild and easy to control, and the apparatus cost, energy cost, and hazards of production can also be reduced.

In a preferred embodiment, the arabinonucleoside includes, but is not limited to, 2,6-Diamino-9-(beta-D-arabinofuranosyl)purine,
nelarabine(9-(β-D-arabinofuranosyl)-6-methoxy-9H-purin-2-amine), 2-chloroadeninearabinoside, or fludarabine(9-β-D-arabinofuranose-2-fluoroadenosine).

A second typical implementation of the present application provides a composition. The composition includes a purine nucleoside phosphorylase and any one of the following: a uracil nucleoside phosphorylase or a pyrimidine nucleoside phosphorylase. The uracil nucleoside phosphorylase includes UP, or a protein that is of more than 80% identity to the UP and has a function same as the UP, and the UP is a protein shown in SEQ ID NO: 1; the pyrimidine nucleoside phosphorylase includes PyNP, or a protein that is of more than 80% identity to the PyNP and has a function same as the PyNP, and the PyNP is a protein shown in SEQ ID NO: 2; and the purine nucleoside phosphorylase includes PNP, or a protein that is of more than 80% identity to the PNP and has a function same as the PNP, and the PNP is a protein shown in SEQ ID NO: 3.

In a preferred embodiment, one or more of the purine nucleoside phosphorylase, the uracil nucleoside phosphorylase or the pyrimidine nucleoside phosphorylase are purified proteins, crude enzyme liquid or immobilized enzymes.

In a preferred embodiment, the composition further includes a 1-beta-D-Arabinofuranosyluracil, a substrate base, and a substrate phosphate. The substrate phosphate is not limited to a specific type, and includes, but is not limited to, phosphates commonly used in the prior art, including, but not limited to, one or more of sodium monohydrogen phosphate, sodium dihydrogen phosphate, potassium monohydrogen phosphate, or potassium dihydrogen phosphate, or mixtures such as phosphate buffer common in the prior art.

In a preferred embodiment, the substrate base is a base shown in Formula I, where R₁ is selected from -NH₂, =O, -OMe or -Cl, and R₂ is selected from -H, -NH₂, -Cl or -F; and preferably, the substrate base includes 2,6-diaminopurine, 2-amino-6-methoxypurine, 2-chloroadenine or 2-fluoroadenine.

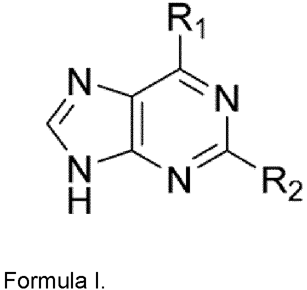

In a preferred embodiment, a concentration of the 1-beta-D-Arabinofuranosyluracil is 2-320 mM, a concentration of the substrate base is 1-200 mM, and a concentration of the substrate phosphate is 1-100 mM.

The concentration of the 1-beta-D-Arabinofuranosyluracil includes 2 mM, 10 mM, 20 mM, 50 mM, 100 mM, 200 mM, and can reach 320mM or more; and the concentration of the substrate base includes 1 mM, 2 mM, 10 mM, 20 mM, 50 mM, 100 mM, and can reach 200 mM or more.

The uracil nucleoside phosphorylase is a protein that is derived from *Trypanosoma cruzi,* shown in SEQ ID NO: 1, and named UP. The pyrimidine nucleoside phosphorylase is a protein that is derived from *Thermus thermophilus,* shown in SEQ ID NO: 2, and named PyNP. The purine nucleoside phosphorylase is a protein that is derived from *Geobacillus stearothermophilus,* shown in SEQ ID NO: 3, and named PNP. The composition can catalyze the substrate base and the 1-beta-D-Arabinofuranosyluracil to undergo a reaction, so as to generate the arabinonucleoside. The enzymes in the composition can be each independently selected from forms such as purified proteins, crude enzyme liquid or immobilized enzymes, and can all achieve a catalysis effect.

The composition may also be prepared in the form of a product such as a kit.

The beneficial effects of the present application are further described in detail below with reference to specific embodiments.

### Embodiment 1

### 1. Strain construction

The uracil nucleoside phosphorylase used in the present application was derived from *Trypanosoma cruzi* (which was named UP, with an amino acid sequence shown in SEQ ID NO: 1). The pyrimidine nucleoside phosphorylase was derived from *Thermus thermophilus* (which was named PyNP, with an amino acid sequence shown in SEQ ID NO: 2). The purine nucleoside phosphorylase was derived from *Geobacillus stearothermophilus* (which was named PNP, with an amino acid sequence shown in SEQ ID NO: 3). After codon optimization, DNA sequences encoding the three enzymes were obtained. The DNA sequence for coding the UP was SEQ ID NO: 4; the DNA sequence for coding the PyNP was SEQ ID NO: 5; and the DNA sequence for coding the PNP was SEQ ID NO: 6. The three DNA sequences were respectively cloned onto an expression vector pET28a(+). Obtained plasmids were transferred into *Escherichia coli* BL21(DE3) host competence, so as to obtain monoclonal strains.
SEQ ID NO: 1: 
MSKATHTHGLMKDPDLPVTADGVTYHLNCKSDQLADRILLVGDPGRVDTVAACFDAGSVRF
RSENREIFIATGTYKGTPVSVLSTGMGTDNVEIVINEIHLLKEYDFERCSWRPRVGDADVPPGAKVF
DPSSVKMIRVGTCGSPFPDMPLAALAITRHVIGMDNTCLYYKVKEQMQKSKDLQEVQRVVKEQTS
LGAIDVYATKAHPAITNGIVAACKAFNDNLPAGATEQLYVVGATATASGFYACQGRAVGRFREHL
LVPKLVEELSKVQFDVSEGKEIVANIEMETSAVCFLSHLLGYQAGSMCVVVAKRAGAEHLFTTSEQ
SGQALKNAIQIALEAIVAVG.
SEQ ID NO: 2: 
MNPVAFIREKREGKKHRREDLEAFLLGYLRDEVPDYQVSAWLMAAFLRGLDPEETLWLTETM
ARSGKVLDLSGLPHPVDKHSSGGVGDKVSLVVGPILAASGCTFAKMSGRGLAHTGGTIDKLESVPG
WRGEMTEAEFLERARRVGLVIAAQSPDLAPLDGKLYALRDVTATVESVPLIASSIMSKKLAAGARSI
VLDVKVGRGAFMKTLEEARLLAKTMVAIGQGAGRRVRALLTSMEAPLGRAVGNAIEVREAIEALK
GEGPGDLLEVALALAEEALRLEGLDPALARKALEGGAALEKFRAFLEAQGGDPRAVEDFSLLPLAE
EHPLRAEREGVVREVDAYKVGLAVLALGGGRKRKGEPIDHGVGVYLLKKPGDRVERGEALALVY
HRRRGLEEALGHLREAYALGEEAHPAPLVLEAI.
SEQ ID NO: 3: 
MSVHIGAKEHEIADKILLPGDPLRAKYIAETFLEGATCYNQVRGMLGFTGTYKGHRISVQGTG
MGVPSISIYITELMQSYNVQTLIRVGTCGAIQKDVKVRDVILAMTSSTDSQMNRMTFGGIDYAPTAN
FDLLKTAYEIGKEKGLQLKVGSVFTADMFYNENAQFEKLARYGVLAVEMETTALYTLAAKFGRKA
LSVLTVSDHILTGEETTAEERQTTFNEMIEVALETAIRQ.
SEQ ID NO: 4: 
atgagcaaagcgacccatacccacggcctgatgaaagatccggatctgccggtgacggcggatggcgttacctatcatctgaactgcaaaagcgatc
agctggcggatcgcattctgctggtgggcgatccgggtcgcgttgataccgtagcggcatgttttgatgcgggcagcgttcgctttcgcagcgaaaaccgcg
 
aaatttttattgcgaccggcacctataaaggcaccccggtgtcagtgctgagcaccggtatgggtactgataatgtggaaattgtgattaacgagattcacctgct
gaaggagtatgacttcgagcgctgcagctggcgcccgcgtgttggtgatgcggatgttcctcctggtgcgaaagtgtttgatccgagcagcgtgaaaatgatt
cgcgtgggcacctgcggcagcccgtttcctgatatgcctctggcggcgctggcgattacccgccatgttattggcatggataacacctgcctgtattataaagtg
aaagagcagatgcagaagagcaaggatctgcaggaagtgcagcgcgtggtgaaagaacagaccagcctgggcgcgattgatgtgtatgcgaccaaagcg
catccggcgattaccaacggcattgtggcggcgtgtaaagcgtttaacgataacctgccggcgggcgcgactgaacaattatatgttgttggcgcgaccgcg
accgcgagcggtttttatgcgtgtcaaggtcgcgcggtgggccgctttcgtgaacatttactggttccgaaactggtggaagaactgagcaaagtgcagtttga
tgtgagcgaaggcaaagaaattgtggcgaacattgaaatggaaaccagcgcggtgtgctttctgagccatctgctgggctatcaggcgggctcaatgtgcgtt
gttgttgcgaaacgcgcgggcgcggaacatctgtttaccaccagcgaacagagcggccaggcgctgaaaaacgcgattcagattgcgctggaagcgattgt
ggcggtgggctaa.
SEQ ID NO: 5: 
atgaacccggtggcgtttattcgcgaaaaacgcgaaggcaagaaacatcgccgcgaggatctggaagcgtttctgctgggctatctgcgcgatgaag
tgccggattatcaggtgagcgcgtggctgatggcggcgtttttacgcggcttagatccggaagaaaccctgtggctgaccgaaacgatggcccgcagcggta
aagtgctggatctgagcggtctgccgcatccggttgataaacatagcagcggcggcgtgggcgataaagtgagcttagtggtgggcccgattctggcggcg
agcggttgtacctttgcgaaaatgtcgggccgcggcctggcgcataccggtggtaccattgataaactggaaagcgtgccgggctggcgcggcgaaatgac
cgaagcagaatttctggaacgcgcccgtcgcgttggtctggttattgcggcgcaaagcccggatttagcgccgttagatggcaaactgtatgcgctgcgcgat
gtgaccgcgaccgttgaatcagttccgctgattgcgagcagcattatgagcaagaaactggcggcgggcgcccgtagcattgtgttagatgttaaagtgggcc
gcggcgcgtttatgaaaaccctggaagaagcgcgtctgctggcgaaaacgatggtggcgattggccagggcgcgggccgtcgtgttcgtgcgttattaacc
agcatggaagcgcctctgggtcgtgcggttggcaatgcgattgaagtgcgtgaagcgattgaagcgctgaaaggcgaaggcccgggcgatttattagaagt
ggcgctggcgttagcggaagaagcgctgcgtttagaaggcttagatccggcgctggcgcgtaaagcgttagaaggtggcgcggcgctggaaaaatttcgc
gcgtttctggaagcgcagggcggcgatccgcgtgcagttgaagattttagcctgctgccgctggcggaagaacatccgttacgtgcggaacgcgaaggcgt
ggttcgcgaagttgatgcgtataaagtgggcctggcggtgctggcgttaggcggtggtcgtaaacgtaaaggtgaaccgattgatcatggcgtgggcgtgtat
ctgctgaagaaaccgggcgatcgcgtggaacgcggcgaagcgttagcgttagtgtatcatcgccgccgcggcttagaagaagcgctgggtcatttacgcga
agcgtatgcgctgggcgaagaagcgcatccggcgcctctggttttagaagcgatttaa.
SEQ ID NO: 6:

atgagcgtgcatattggcgcgaaagaacatgaaattgcggataaaattctgctgccgggcgatccgttacgcgcgaaatatattgcggaaacctttctg
gaaggcgcgacctgctataaccaggtgcgcggcatgctgggctttaccggcacctataaaggccatcgcattagcgtgcagggcaccggcatgggcgttcc
tagcatttcaatttatattaccgaactgatgcagagctataacgtgcagaccctgattcgcgtgggcacctgcggtgcgattcaaaaagatgtgaaagtgcgcga
tgtgattctggcgatgaccagcagcaccgatagccagatgaaccgcatgacctttggcggcattgattatgcgccgaccgcgaactttgatctgctgaaaacc
gcgtatgaaattggcaaagaaaaaggcctgcagctgaaagtgggcagcgtgtttaccgcggatatgttttataacgaaaacgcgcagtttgagaaactggccc
gctatggcgtgctggcggttgaaatggaaaccaccgcgctgtataccctggcggcgaaatttggccgcaaagcgctgagcgtgctgaccgtgagcgatcat
attctgaccggcgaagaaaccaccgcggaagaacgccagaccacctttaacgaaatgattgaagtggcgctggaaaccgcgattcgccagtaa.

### 2. Protein expression

The *Escherichia coli* strains expressing the UP, PyNP, and PNP were respectively inoculated into test tubes, cultured at 37 °C for 16 h, and then inoculated into 2L shake flasks containing 500 mL of Luria-Bertani medium according to a 1% inoculation amount; culture was performed at 37 °C until OD600 was 0.6, methanesulfonic anhydride with a final concentration being 0.1 M was added to induce protein expression; and culture was performed at 20 °C for 18 h. The cultured bacterial fluid was centrifuged at 7000 rpm for 10 min, and then bacterial cells were collected for later use.

### 3. Enzyme liquid preparation

0.1 g of bacterial sludge was weighed, 1 mL of a potassium phosphate buffer with pH being 7.5 was added, after shaking and well mixing, a sonicator was used to crush bacterial suspension for 5 min with power of 30%.

### 4. Detection method

HPLC assay method: chromatographic column: Agilent Eclipse plus C18, 4.6×100 mm, 3.5 µm; a mobile phase was water and acetonitrile, with a flow rate of 1.5 mL/min and a temperature of 40 °C; and UV: 254 nm.

### Embodiment 2: Synthesis of 2,6-Diamino-9-(beta-D-arabinofuranosyl)purine

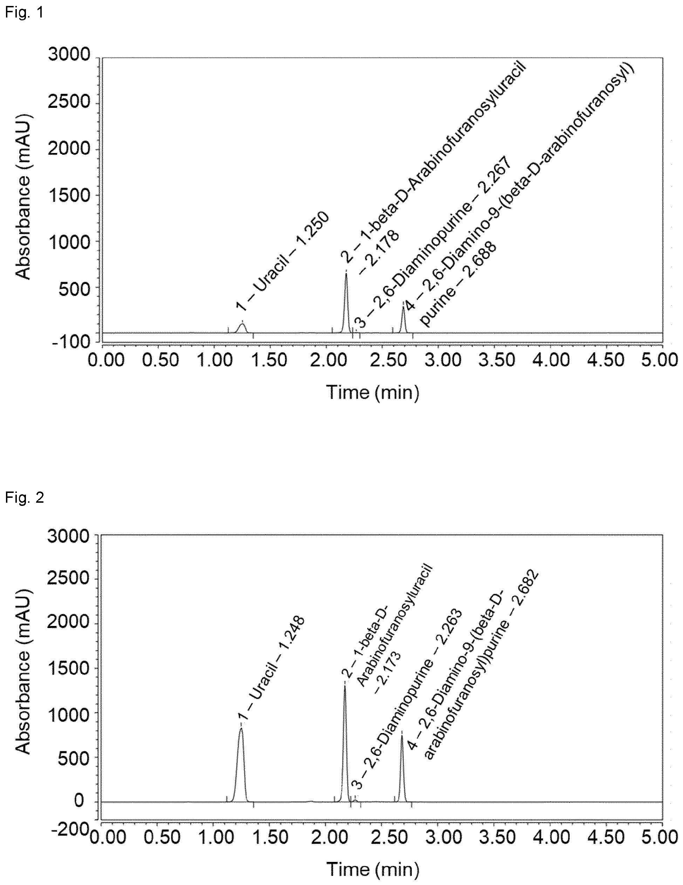

1 mL of a reaction system was prepared in 2 mM of a phosphate buffer (pH 7.5), including 80 mM of 1-beta-D-Arabinofuranosyluracil, 20 mM of 2,6-Diaminopurine, UP enzyme liquid prepared by 4.88 mg of bacterial sludge, and PNP enzyme liquid prepared by 6.01 mg of bacterial sludge, a reaction was performed for 16 h at 60 °C, as shown in Fig. 1, a conversion rate of the 2,6-Diaminopurine was 98.78%.

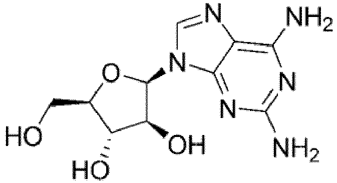

### 2,6-Diamino-9-(beta-D-arabinofuranosyl)purine

### Embodiment 3: Amplification reaction of synthesis of 2,6-Diamino-9-(beta-D-arabinofuranosyl)purine

1 mL of a reaction system was prepared in 2 mM of a phosphate buffer (pH 7.5), including 320 mM of 1-beta-D-Arabinofuranosyluracil, 200 mM of 2,6-Diaminopurine, UP enzyme liquid prepared by 19.54 mg of bacterial sludge, and PNP enzyme liquid prepared by 60.06 mg of bacterial sludge, a reaction was performed for 6 h at 60 °C, a conversion rate of the 2,6-Diaminopurine was 89.74%. When the reaction proceeded for 16 hours(as shown in Fig. 2), the conversion rate of the 2,6-Diaminopurine was 96.74%, and a yield of the 2,6-Diamino-9-(beta-D-arabinofuranosyl)purine was 54.61 g/L.

### Embodiment 4: Synthesis of Nelarabine (9-(β-D-arabinofuranosyl)-6-methoxy-9H-purin-2-amine)

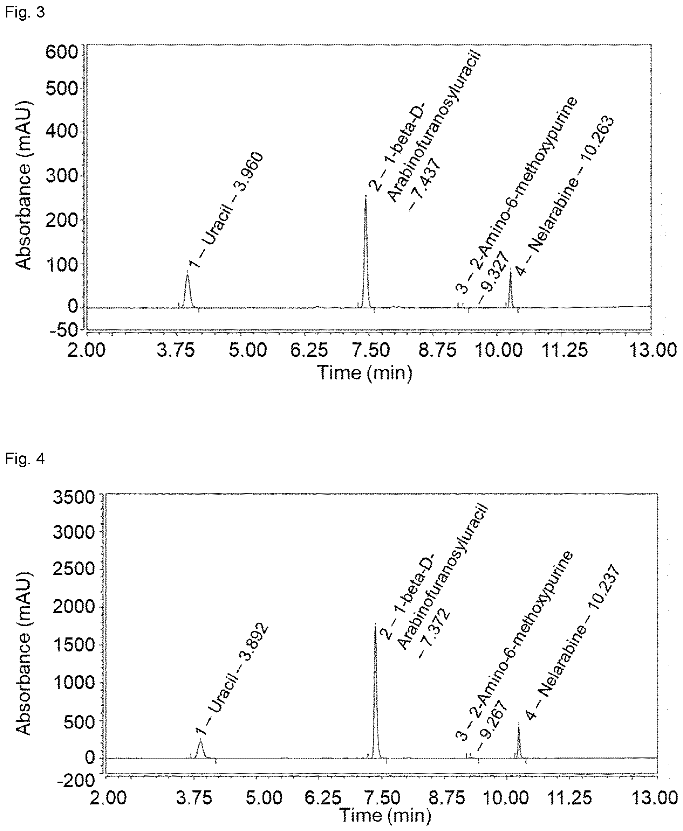

1 mL of a reaction system was prepared in 2 mM of a phosphate buffer (pH 7.5), including 4 mM of 1-beta-D-Arabinofuranosyluracil, 1 mM of 2-Amino-6-methoxypurine, UP enzyme liquid prepared by 0.24 mg of bacterial sludge, and PNP enzyme liquid prepared by 0.33 mg of bacterial sludge, a reaction was performed for 6 h at 70 °C, as shown in Fig. 3, a conversion rate of the 2-Amino-6-methoxypurine was 98.48%.

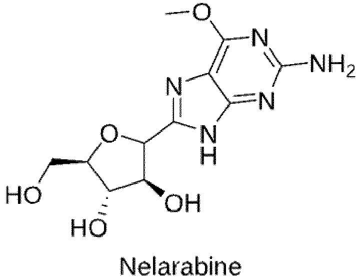

### Embodiment 5: Amplification reaction of Nelarabine (9-(β-D-arabinofuranosyl)-6-methoxy-9H-purin-2-amine)

1 mL of a reaction system was preapred in 2 mM of a phosphate buffer (pH 7.5), including 320 mM of 1-beta-D-Arabinofuranosyluracil, 80 mM of 2-Amino-6-methoxypurine, UP enzyme liquid prepared by 19.54 mg of bacterial sludge, and PNP enzyme liquid prepared by 26.42 mg of bacterial sludge, a reaction was performed for 16 h at 70 °C, a conversion rate of 6-Methoxypurine was 74.82%. The reaction was performed for 40 h, a conversion rate of the 2-Amino-6-methoxypurine was 94.96%, as shown in Fig. 4, a yield of the Nelarabine was 22.58 g/L.

### Embodiment 6: Synthesis of 2-chloroadeninearabinoside

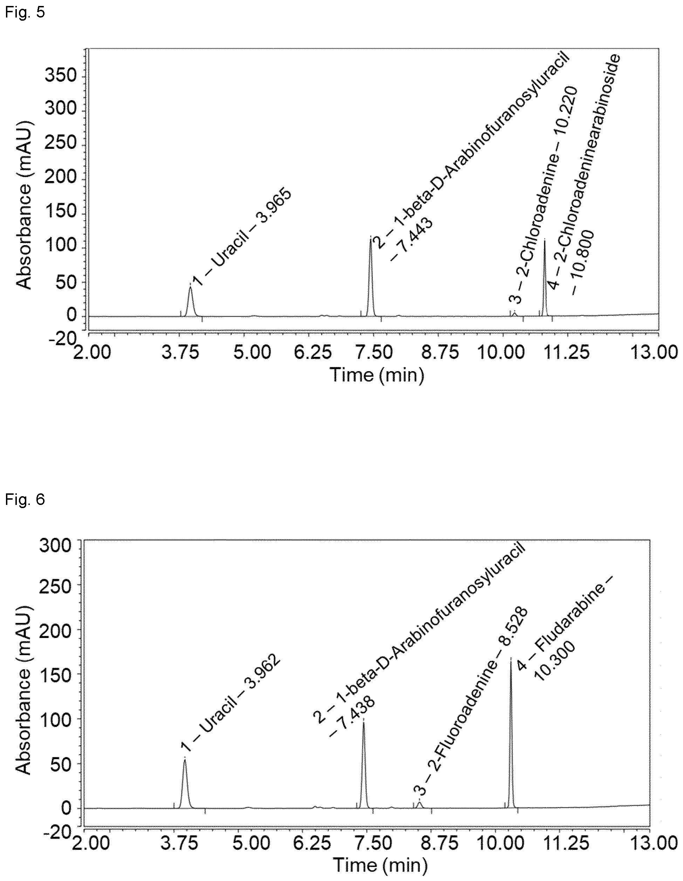

1 mL of a reaction system was prepared in 2 mM of a phosphate buffer (pH 7.5), including 2 mM of 1-beta-D-Arabinofuranosyluracil, 1 mM of 2-Chloroadenine, UP enzyme liquid prepared by 0.12 mg of bacterial sludge, and PNP enzyme liquid prepared by a proper amount of bacterial sludge, and a reaction was performed for 20 h at 70 °C. As shown in Fig. 5, a conversion rate of the 2-Chloroadenine was 93.97%.

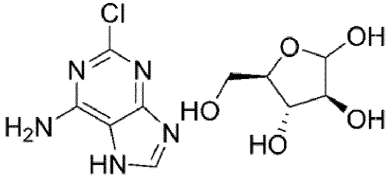

### 2-chloroadeninearabinoside

### Embodiment 7: Synthesis of Fludarabine (9-β-D-arabinofuranose-2-fluoroadenosine)

1 mL of a reaction system was prepared in 2 mM of a phosphate buffer (pH 7.5), including 2 mM of 1-beta-D-Arabinofuranosyluracil, 1 mM of a purine analogue 2-Fluoroadenine, UP enzyme liquid prepared by 0.12 mg of bacterial sludge, and PNP enzyme liquid prepared by a proper amount of bacterial sludge, and a reaction was performed for 20 h at 70 °C. As shown in Fig. 6, a conversion rate of the 2-Fluoroadenine was 92.51%.

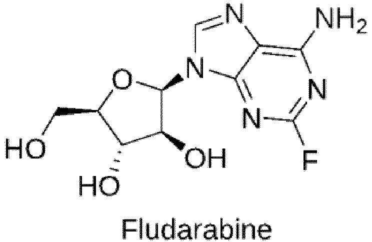

From the above descriptions, it might be seen that the embodiments of the present application achieved the following technical effects. In the present application, with the purine nucleoside phosphorylase, and the pyrimidine nucleoside phosphorylase or the uracil nucleoside phosphorylase, the 1-beta-D-Arabinofuranosyluracil and the substrate base could be used as substrates, a target product arabinonucleoside was prepared through one-pot biosynthesis, and operations of midway material replenishment or intermediate product purification and re-feeding, etc., were not required. Compared to the two-step biosynthesis in the prior art, the operations were simpler, and the time required by the reaction was short. Compared to one-pot biosynthesis in the prior art, a reaction rate was faster, a conversion rate of the substrate was higher, a higher substrate concentration could be tolerated, and large scale industrial production could be realized within a shorter reaction time. Compared to chemical synthesis, the reaction was milder in conditions required, simple in process, low in cost, and environmentally friendly.

The above are only the preferred embodiments of the present application and are not intended to limit the present application. For those skilled in the art, the present application may have various modifications and variations. Any modifications, equivalent replacements, improvements and the like made within the spirit and principle of the present application all fall within the scope of protection of the present application.

## Claims

1. A method for one-pot biosynthesis of an arabinonucleoside, comprising:
mixing a substrate, a purine nucleoside phosphorylase and any one of the following together: a uracil nucleoside phosphorylase or a pyrimidine nucleoside phosphorylase, and, then performing the one-pot biosynthesis, and directly obtaining the arabinonucleoside, wherein
the uracil nucleoside phosphorylase comprises UP, and the UP is a protein shown as SEQ ID NO: 1; or a protein that is of more than 80% identity with the UP and has the same function;
the pyrimidine nucleoside phosphorylase comprises PyNP, and the PyNP is a protein shown as SEQ ID NO: 2; or a protein that is of more than 80% identity with the PyNP and has the same function;
the purine nucleoside phosphorylase comprises PNP, and the PNP is a protein shown as SEQ ID NO: 3; or a protein that is of more than 80% identity with the PNP and has the same function; and
the substrate comprises a 1-beta-D-Arabinofuranosyluracil, a substrate base and a substrate phosphate.

2. The method according to claim 1, comprising:
catalyzing the 1-beta-D-Arabinofuranosyluracil and the substrate phosphate with the uracil nucleoside phosphorylase or the pyrimidine nucleoside phosphorylase to generate an arabinose-1-phosphate and a free base; and
catalyzing the arabinose-1-phosphate with the purine nucleoside phosphorylase, and substituting a phosphate group on the arabinose-1-phosphate with the substrate base, so as to obtain the arabinonucleoside.

3. The method according to claim 1, wherein the substrate base is a base shown in a formula I, wherein R₁ is selected from -NH₂, =O, -OMe or -Cl, and R₂ is selected from -H, -NH₂, -Cl or -F; 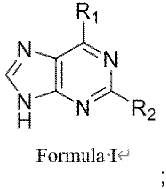 and
preferably, the substrate base comprises 2,6-diaminopurine, 2-amino-6-methoxypurine, 2-chloroadenine or 2-fluoroadenine.

4. The method according to claim 1, wherein one or more of the purine nucleoside phosphorylase, the uracil nucleoside phosphorylase or the pyrimidine nucleoside phosphorylase are purified proteins, crude enzyme liquid or immobilized enzymes.

5. The method according to claim 1, wherein a catalytic time for the one-pot biosynthesis is 4-20h;
preferably, a catalytic temperature of one-pot biosynthesis is 50-80°C, more preferably, 60-70°C; and
preferably, a concentration of the 1-beta-D-Arabinofuranosyluracil is 2-320 mM, a concentration of the substrate base is 1-200 mM, and a concentration of the substrate phosphate is 1-100 mM.

6. The method according to claim 1, wherein the arabinonucleoside comprises 2,6-Diamino-9-(beta-D-arabinofuranosyl)purine, nelarabine, 2-chloroadeninearabinoside, or fludarabine; and
preferably, the substrate phosphate comprises one or more of sodium monohydrogen phosphate, sodium dihydrogen phosphate, potassium monohydrogen phosphate, or potassium dihydrogen phosphate.

7. A composition, comprising a purine nucleoside phosphorylase and any one of the following: a uracil nucleoside phosphorylase or a pyrimidine nucleoside phosphorylase;
the uracil nucleoside phosphorylase comprises UP and the UP is a protein shown as SEQ ID NO: 1, or a protein that is of more than 80% identity with the UP and has the same function;
the pyrimidine nucleoside phosphorylase comprises PyNP, and the PyNP is a protein shown as SEQ ID NO: 2, or a protein that is of more than 80% identity with the PyNP and has the same function; and
the purine nucleoside phosphorylase comprises PNP, and the PNP is a protein shown as SEQ ID NO: 3, or a protein that is of more than 80% identity with the PNP and has the same function.

8. The composition according to claim 7, wherein one or more of the purine nucleoside phosphorylase, the uracil nucleoside phosphorylase or the pyrimidine nucleoside phosphorylase are purified proteins, crude enzyme liquid or immobilized enzymes.

9. The composition according to claim 7, further comprising a 1-beta-D-Arabinofuranosyluracil, a substrate base and a substrate phosphate.

10. The composition according to claim 9, wherein the substrate base is a base shown in a formula I, wherein R₁ is selected from -NH₂, =O, -OMe or -Cl, and R₂ is selected from -H, -NH₂, -Cl or -F; 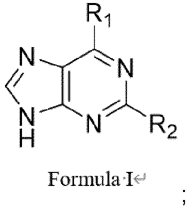
preferably, the substrate base comprises 2,6-diaminopurine, 2-amino-6-methoxypurine, 2-chloroadenine or 2-fluoroadenine;
preferably, the substrate phosphate comprises one or more of sodium monohydrogen phosphate, sodium dihydrogen phosphate, potassium monohydrogen phosphate, or potassium dihydrogen phosphate; and
preferably, a concentration of the 1-beta-D-Arabinofuranosyluracil is 2-320 mM, a concentration of the substrate base is 1-200 mM, and a concentration of the substrate phosphate is 1-100 mM.
